# EUROPÄISCHE PATENTSCHRIFT

(11) **EP 2 415 492 B1**
(45) Veröffentlichungstag und Bekanntmachung des Hinweises auf die Patenterteilung: **13.11.2013**
(21) Anmeldenummer: 11005501.9
(22) Anmeldetag: 06.07.2011
(51) Int. Cl.: A61M 3/02

(54) **Handstückeinheit für eine Einweg-Spüldüseneinheit zur Reinigung und/oder Spülung von Operationswunden**
Handpiece unit for a disposable flushing jet unit for cleaning and/or flushing operation wounds
Pièce de prise en main d'un gicleur unidirectionnel pour nettoyage et/ou rinçage de blessures chirurgicales

(30) Priorität: 03.08.2010 DE 102010033240
(43) Veröffentlichungstag der Anmeldung: 08.02.2012
(73) Patentinhaber: Schwarz, Volker A., 94377 Steinach (DE)
(72) Erfinder: Schwarz, Volker A., 94377 Steinach (DE); Gunzl, Andreas, 94136 Thyrnau (DE)
(74) Vertreter: Graf Glück Kritzenberger

(56) Entgegenhaltungen:
- WO-A1-2006/040273
- WO-A2-2010/016089
- US-A- 5 470 305
- US-A1- 2003 036 723
- US-A1- 2009 281 454

## Beschreibung

Die Erfindung betrifft eine Handstückeinheit für eine Einweg-Spüldüseneinheit zur Reinigung und/oder Spülung von Operationswunden gemäß dem Oberbegriff des Patentanspruches 1.

Spülanordnungen zur Reinigung und/oder Spülung von Operationswunden sind hinlänglich aus dem Stand der Technik bekannt. Derartige Spülanordnungen werden im Stand der Technik auch als "Pulse Lavage" Systeme bezeichnet, welche neben der Reinigung verschmutzter Wunden auch zu effektiven intra-operativen Wundspülung, zur Vorbereitung von lmplantatelager bei Operation, zur Kühlung im Operationsbereich sowie in der septischen Chirurgie Anwendung finden. Hierdurch können eine Vielzahl von Arbeitsgängen in der operativen Medizin bedeutend vereinfacht, beschleunigt und vor allem in der Effektivität verbessert werden.

Derartige Spülanordnungen bestehen im Wesentlichen aus einer Handstückeinheit und einem Einweg-Spüldüsenset bzw. einer Einweg-Spüldüseneinheit. Die Einweg-Spüldüseneinheit besteht aus einem Grundkörper und einem vorzugsweise mehrteiligen Spülrohr mit einer Spritzdüse, an dessen freiem Ende beispielsweise ein Spritzschutzschild vorgesehen sein kann. Der Grundkörper wird an der Handstückeinheit befestigt, welche vorzugsweise ein mehrteiliges Gehäuse mit einer revolver- bzw. pistolengriffartigen Querschnittsform aufweist.

Zur Erzeugung einer Pumpwirkung ist im Grundkörper der Einweg-Spüldüseneinheit eine Pumpenmechanik mit einer Pumpmembran vorgesehen, welche über eine in der Handstückeinheit vorgesehene Antriebsmechanik in Schwingung gebracht wird. Hierbei wird durch die in der Handstückeinheit vorgesehene Antriebsmechanik eine pulsierende Bewegung im Bereich des Anschlusses des Grundkörpers erzeugt, welche die dort hineinreichende Pumpmembran mit einer vorgegebenen Frequenz ausgelenkt und damit eine Schwingung der Pumpmembran erzeugt. Durch die Schwingung der Pumpmembran wird in Verbindung mit der Pumpenmechanik der Einweg-Spüldüseneinheit eine Pumpwirkung erzeugt, über welche eine Spül- bzw. Reinigungsflüssigkeit von einer externen Quelle über ein mit dem Grundkörper verbundenes Schlauchsystem in das Spülrohr eingeführt und über dieses in die jeweilige Operationswunde eingebracht wird.

Bekannte Handstückeinheiten weisen hierfür eine druckluftbetriebene Motoreinheit auf, die in dem vorzugsweise mehrteiligen Gehäuse aufgenommen ist. Über einen sperrigen Druckluftschlauch wird die für den Betrieb der Motoreinheit erforderliche Druckluft an die Handstückeinheit geführt. Derartige druckluftbetriebenen Handstückeinheiten werden mit leistungsfähigen druckluftbetriebenen Motoreinheiten ausgestattet, welche bei einem Betriebsdruck von 5 - 7 bar eine optimale Reinigung und/oder Spülung der Operationswunde gewährleisten. Nachteilig erschwert jedoch der hierzu erforderliche sperrige Druckluftschlauch die Handlichkeit der Handstückeinheit. Auch ist die Spülleistung bekannter druckluftbetriebener Handstückeinheiten nicht individuell an die jeweiligen Operationsbedürfnisse anpassbar.

Ferner sind als Einwegartikel ausgebildete, kostengünstige Handstückeinheiten bekannt, welche nach einem einmaligen Gebrauch im Operationssaal entsorgt werden. Diese weisen eine in der Handstückeinheit aufgenommene Elektromotoreinheit mit jedoch geringer Leistung auf und verfügen über eine in der Handstückeinheit aufgenommene, interne elektrische Energieversorgung, vorzugsweise in Form von mehreren Batterien oder Akkus. Die Spülleistung derartiger als Einwegartikel ausgebildeter, kostengünstiger Handstückeinheiten ist jedoch beschränkt und häufig unzureichend. Auch werden besonders nachteilig die in der Handstückeinheit aufgenommenen Batterien oder Akkus, beispielsweise des Typs AA oder AAA bereits nach einer kurzen Einsatzdauer von ca. 3 - 5 Minuten entsorgt.

Beispiele solcher Handstückeinheiten werden z.B. in US 2003/036723 A1 und US 5 470 305 beschrieben.

Ausgehend hiervon liegt der Erfindung die Aufgabe zugrunde, eine Handstückeinheit für eine Einweg-Spüldüseneinheit zur Reinigung und/oder Spülung von Operationswunden anzugeben, welches eine verbesserte Handhabbarkeit bei hoher Spülleistung aufweist. Die Aufgabe wird ausgehend von den Merkmalen des Oberbegriffes des Patentanspruches 1 durch dessen kennzeichnende Merkmale gelöst. Der wesentliche Aspekt der erfindungsgemäßen Handstückeinheit ist darin zu sehen, dass die Handstückeinheit eine zweite Anschlusseinheit zum Anschluss einer externen elektrischen Energieversorgung aufweist, über welche die im Gehäuse integrierte Elektromotor mit vorzugsweise hoher Leistung mit elektrischer Energie versorgt wird. Der Elektromotor wird vorzugsweise über ein externes Netzteil, welches über ein Verbindungskabel mit dem Handstück über eine Steckverbindung verbunden ist, mit elektrischer Energie versorgt. Durch das Entfallen des Druckluftschlauches und erfindungsgemäße Energieversorgung über ein dünnes, elastisches Verbindungskabels eines Netzteils wird die Handhabbarkeit wesentlich verbessert, ohne jedoch die mittels der Elektromotoreinheit erzeugte Leistung zu reduzieren. Hierbei kann die zweite Anschlusseinheit als Steckeraufnahmeeinheit ausgebildet sein, an der beispielsweise zumindest zwei unterschiedliche Betriebsspannungen anliegen können.

Weiterhin vorteilhaft ist im Gehäuse der Handstückeinheit eine Steuereinheit vorgesehen, die zum Betrieb der Elektromotoreinheit in zumindest zwei unterschiedlichen Leistungsstufen und/oder zwei unterschiedlichen Betriebsarten, insbesondere Intervall- oder Dauerbetrieb, ausgebildet ist. Die Steuereinheit ist beispielsweise zur Steuerung der Drehzahl der Elektromotoreinheit vorgesehen, und zwar im Bereich zwischen 1200 U/min bis 5000 U/min. Beispielsweise kann eine Leistungsstufe für eine reduzierte Pumpleistung vorgesehen sein, welche bei Behandlung von sensiblen Gewebe- oder Weichteilen im Bereich der plastischen Chirurgie ausgewählt werden kann. Alternativ oder zusätzlich kann Dauerbetrieb eingestellt werden, wodurch die Handstückeinheit auch ohne Betätigung des Auslösers betrieben wird. Insbesondere bei infizierten (septischen) Operationen bietet dies gegenüber herkömmlichen Handstückeinheiten einen besonderen Vorteil, und zwar kann dadurch die Spülmenge erhöht werden und der Operateur hat eine zusätzliche Hand frei.

Die Antriebsmechanik ist zur Umsetzung einer Drehbewegung der Elektromotoreinheit in eine pulsierende Schubbewegung ausgebildet und umfasst vorzugsweise zumindest ein stößel- oder pilzartig ausgebildetes Schubelement, ein Schubstangenelement und ein vorzugsweise zylinderförmig ausgebildetes Antriebselement, wobei das Antriebselement mit der Elektromotoreinheit in Wirkverbindung steht. Das Antriebselement weist hierbei einen Antriebsstift auf, der in eine im Schubstangenelement vorgesehene Aufnahmebohrung eingreift.

Besonders vorteilhaft ist die Handstückeinheit re-sterilisierbar ausgebildet

Die Handstückeinheit weist zumindest ein an die Steuereinheit angeschlossenes Schaltmittel auf, welches in Wirkverbindung mit einem Betätigungsmittel steht. Ferner kann zumindest eine Anzeigeneinheit zur Anzeige der Betriebsart und/oder der Leistungsstufe der Elektromotoreinheit vorgesehen sein.

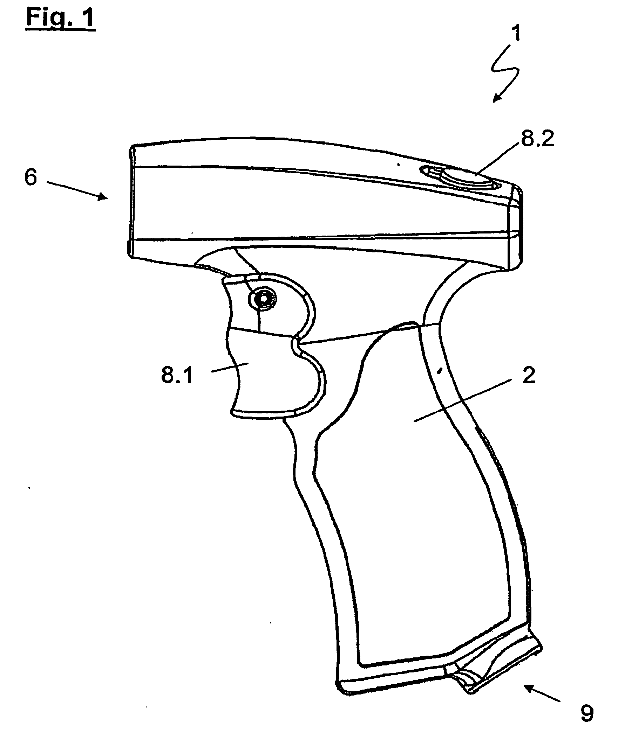

Die Erfindung soll nachfolgend anhand von Ausführungsbeispielen im Zusammenhang mit den Figuren näher erläutert werden. Zudem ergeben sich Weiterbildungen, Vorteile und Anwendungsmöglichkeiten der Erfindung auch aus der nachfolgenden Beschreibung der Ausführungsbeispiele und aus der Figuren. Dabei sind alle beschriebenen und/oder bildlich dargestellten Merkmale für sich oder in beliebiger Kombination grundsätzlich Gegenstand der Erfindung, unabhängig von ihrer Zusammenfassung in den Ansprüchen oder deren Rückbeziehung. Auch wird der Inhalt der Ansprüche zu einem Bestandteil der Beschreibung gemacht. Es wird aber ausdrücklich darauf hingewiesen, dass die Erfindung keinesfalls auf die angegebenen Ausführungsbeispiele beschränkt sein soll. Es zeigen
- Fig. 1: beispielhaft eine schematische Seitenansicht einer erfindungsgemäßen Handstückeinheit,

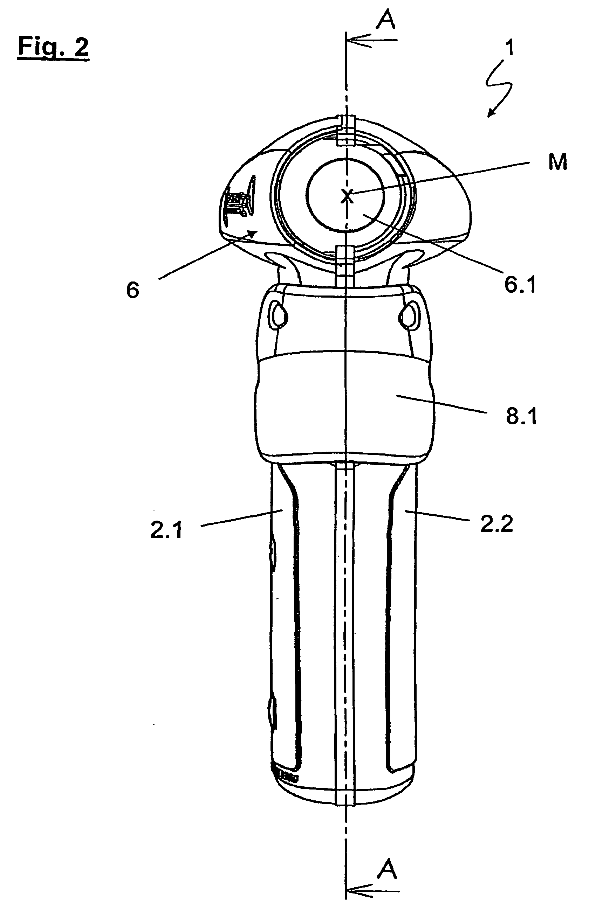

- Fig. 2: beispielhaft eine Stirnansicht der erfindungsgemäßen Handstückeinheit,

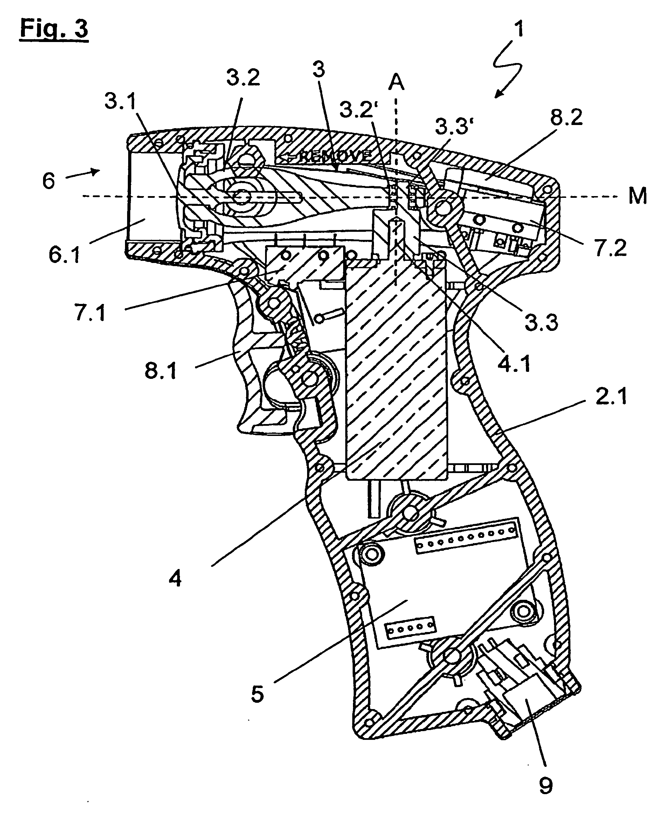

- Fig. 3: beispielhaft einen Längsschnitt entlang der Linie A-A durch die erfindungsgemäßen Handstückeinheit gemäß Figur 2.

In den Figuren wird mit 1 eine erfindungsgemäße Handstückeinheit zum Betrieb einer nicht in den Figuren dargestellten Einweg-Spüldüseneinheit zur Reinigung und/oder Spülung von Operationswunden bezeichnet.

Die Handstückeinheit 1 umfasst ein vorzugsweise mehrteiliges Gehäuse 2, welches beispielsweise aus Metall oder Kunststoff oder einer Kombination vorgenannter Materialien hergestellt ist. Das Gehäuse 2 ist in vorliegendem Ausführungsbeispiel zweiteilig ausgebildet und weist eine erste Gehäusehälfte 2.1 und eine zweite Gehäusehälfte 2.2 auf, die jeweils schalenartig ausgebildet sind. Die erste und zweite Gehäusehälfte 2.1, 2.2 schließen einen Aufnahmeraum ein, in dem die unterschiedlichen Bauteile der Handstückeinheit 1 aufgenommen sind. Das Gehäuse 2 besitzt in einer bevorzugten Ausführungsform eine revolver- bzw. pistolengriffartige Querschnittsform. Ebenso ist das Gehäuse 2 aus einem widerstandsfähigen, insbesondere bruchsicheren Material hergestellt, welches re-sterilisierbar ist.

Im Gehäuse 2 der Handstückeinheit 1 sind zumindest eine Antriebsmechanik 3 und eine mit dieser zusammenwirkende Elektromotoreinheit 4 vorgesehen, wobei zur Ansteuerung der Elektromotoreinheit 4 eine Steuereinheit 5 vorgesehen ist, die vorzugsweise im unteren Griffbereich der Handstückeinheit 1 im Gehäuse 2 aufgenommen ist. Die Antriebsmechanik 3 dagegen ist beispielsweise im oberen Griffbereich der Handstückeinheit 1 vorgesehen.

Die Handstückeinheit 1 verfügt ferner über eine erste Anschlusseinheit 6, welche zur Aufnahme einer Einweg-Spüldüseneinheit ausgebildet ist, und zwar eines hierfür vorgesehenen Anschlussabschnittes des Grundkörpers der Einweg-Spüldüseneinheit. Die erste Anschlusseinheit 6 umfasst eine im Querschnitt kreisförmige Ausnehmung 6.1, welche durch die stirnseitigen freien Enden der erste und zweite Gehäusehälfte 2.1, 2.2 eingeschlossen wird.

Vorzugsweise weist die erste Anschlusseinheit 6 ein Innengewinde auf, über welches der mit einem Außengewinde versehene Anschlussbereich der Einweg-Spüldüseneinheit an der Handstückeinheit 1 befestigt wird. Beispielsweise kann die beschriebene Schraubverbindung in Form eines Bajonettverschlusses realisiert sein.

Der Anschlussbereich der Einweg-Spüldüseneinheit wird hierbei derart in der Ausnehmung 6.1 aufgenommen ist, dass eine im Anschlussbereich der Einweg-Spüldüseneinheit vorgesehene und von außen zugängliche Pumpmembran in Wirkverbindung mit der Antriebsmechanik 3 der Handstückeinheit 1 tritt.

Ferner sind in der Handstückeinheit 1 Schaltmittel vorgesehen, welche mit Betätigungsmittel zusammenwirken, über welche der Benutzer der Handstückeinheit 1 die Betriebsart und/oder die Leistung der Elektromotoreinheit variieren kann.

Im vorliegenden Ausführungsbeispiel sind beispielsweise zumindest erste und zweite Schaltmittel 7.1, 7.2 vorgesehen, welche über im oder am Gehäuse 2 angeordnete erste und zweite Betätigungsmittel 8.1, 8.2 aktivierbar sind. Das erste Betätigungsmittel 8.1 ist beispielsweise mit einem ersten Schaltmittel 7.1 und das zweite Betätigungsmittel 8.2 mit einem zweiten Schaltmittel 7.2 verbunden, wobei das erste und zweite Schaltmittel 7.1, 7.2 an die Steuereinheit 5 angeschlossen sind, welche ihrerseits mit der Elektromotoreinheit 4 in Verbindung steht. Das erste Betätigungsmittel 8.1 ist im vorliegenden Ausführungsbeispiel in Form eines Wippschalters realisiert, über welchen die Handstückeinheit 1 ein- oder ausgeschaltet werden kann. Als zweite Schaltmittel 7.2 sind beispielsweise zwei Schalter an der Oberseite des Gehäuses 2 vorgesehen, welche symmetrisch zur Schnittebene A-A angeordnet sind.

Ferner umfasst die Handstückeinheit 1 zumindest eine zweite Anschlusseinheit 9, welche im unteren Griffbereich der Handstückeinheit 1 bzw. dessen Gehäuse 2 vorgesehen ist. Die zweite Anschlusseinheit 9 dient zum Anschluss einer externen elektrischen Energieversorgung und ist vorzugsweise als Steckanschlusseinheit ausgebildet. Insbesondere können an der Steckanschlusseinheit zumindest zwei unterschiedliche Betriebspannungen anliegen, welche jeweils zum Betrieb der Elektromotoreinheit 4 über die Steuereinheit 5 auswählbar sind.

Über die zweite Anschlusseinheit 9 wird somit der Handstückeinheit 1, die für den Betrieb der Elektromotoreinheit 4 und der Steuereinheit 5 erforderliche elektrische Energie, insbesondere Betriebsspannung zugeführt. Die externe Energiequelle ist vorzugsweise durch ein elektrisches Netzteil gebildet, welches über ein mehradriges elektrisches Verbindungskabel, an dessen freiem Ende ein Steckanschluss vorgesehen ist, mit der zweiten Anschlusseinheit 9 zusammenwirkt. Die Elektromotoreinheit wird hierüber beispielsweise mit einer Betriebsspannung zwischen 4,5 Volt und 17,5 Volt bei einer Stromstärke von 0,5 A bis 0,8 A versorgt.

Die Leistung der Elektromotoreinheit ist beispielsweise einstellbar, und zwar sind zumindest zwei unterschiedliche Leistungsstufen wählbar, bei denen die Drehzahl im Bereich zwischen 1200 U/min bis 5000 U/min liegt. Die Antriebsmechanik 3 und die Elektromotoreinheit 4 wirken hierbei derart zusammen, dass eine pulsierende Schubbewegung im Bereich der ersten Anschlusseinheit 6, insbesondere innerhalb der kreisförmigen Ausnehmung 6.1 erzeugt wird, welche auf die Pumpmembran der Einweg-Spüldüseneinheit einwirkt und diese in eine entsprechende Schwingung versetzt, wodurch beispielsweise eine Pumpwirkung von 5 ml/sec bis 15 ml/sec erzeugt wird.

Die Antriebsmechanik 3 umfasst hierbei ein stößel- oder pilzartig ausgebildetes Schubelement 3.1, welches einen T-förmigen Querschnitt aufweist. Das stößel- oder pilzartige Schubelement 3.1 ist horizontal geführt, und zwar entlang der Mittenachse M der kreisförmigen Ausnehmung 6.1. An dem stößel- oder pilzartigen Schubelement 3.1 liegt ein Schubstangenelement 3.2 an, welches beweglich entlang der Mittenachse M im Gehäuse 2 gelagert ist. In der Stirnseite des Schubstangenelementes 3.2 ist der längliche Teil des stößel- oder pilzartig ausgebildetes Schubelement 3.1 aufgenommen, wobei am gegenüberliegenden Ende des Schubstangenelementes 3.2 eine senkrecht zur Mittenachse M verlaufende Aufnahmebohrung 3.2' vorgesehen, in welcher ein Antriebsstift 3.3' eines Antriebselementes 3.3 eingreift und darin drehbar gelagert ist.

Das Antriebselement 3.3 ist vorzugsweise zylinderförmig ausgebildet und zur Aufnahme einer Antriebswelle 4.1 der Elektromotoreinheit 4 vorgesehen. Die Antriebswelle 4.1 der Elektromotoreinheit 4 wird um die Antriebsachse A angetrieben, welche vorzugsweise senkrecht zu Mittenachse M verläuft. Über die Antriebswelle 4.1 wird das damit in Wirkverbindung stehende kreiszylinderförmige Antriebselement 3.3 in eine Drehbewegung um die Antriebsachse A versetzt. Das Antriebsstiftelement 3.3' des Antriebselementes 3.3 ist hierbei exzentrisch zur Antriebsachse A vorgesehen, so dass die Drehbewegung des Antriebselementes 3.3 in eine pulsierende Schubbewegung des Schubstangenelementes 3.2 entlang der Mittenachse M umgesetzt wird. Somit ist die Antriebsmechanik 3 zur Umsetzung der Drehbewegung der Elektromotoreinheit 4 in eine pulsierende Schubbewegung vorgesehen. Hierbei wird durch die Elektromotoreinheit 4 und die Antriebsmechanik 3 eine pulsierende Schubbewegung zwischen 20 Hz und 70 Hz erzeugt, mit welcher die Pumpmembran der Einweg-Spüldüseneinheit beaufschlagt wird.

In einer bevorzugten Ausführungsform ist mittels der Steuereinheit 5 die Drehzahl der Elektromotoreinheit einstellbar, und zwar im Bereich zwischen 1200 U/min bis 5000 U/min. Vorzugsweise sind zumindest eine erste und zweite Leistungsstufe vorgesehen, welche über die zweiten Schaltmittel 7.2 bzw. die mit diesen verbundenen zweiten Betätigungsmittel 8.2 auswählbar sind. Über die ersten Schaltmittel 7.1 bzw. die ersten Betätigungsmittel 8.1 wird die Elektromotoreinheit in Betrieb genommen, d.h. die Handstückeinheit aktiviert und entsprechend der Spülbetrieb aufgenommen.

In einer weiteren Ausführungsform sind mittels der Steuereinheit 5 unterschiedliche Betriebsarten der Elektromotoreinheit 4 voreinstellbar, und zwar ist beispielsweise die Elektromotoreinheit in Intervall- oder Dauerbetrieb betreibbar. Hierzu wird durch entsprechende Betätigung der zweiten Betätigungsmittel 8.2 an der Oberseite des Gehäuses 2 durch den Nutzer ausgewählt, ob die Handstückeinheit 1 im Dauerbetrieb, d.h. ohne Betätigung der ersten Schaltmittel 8.1 betreibbar sein soll oder im Intervallbetrieb, d.h. abhängig von der Betätigung des ersten Betätigungsmittels 8.1 ein-oder ausgeschaltet wird. Zusätzlich können über dritte Betätigungsmittel und damit verbundene dritte Schaltmittel zwischen zwei Leistungsstufen gewählt werden, wobei bei Auswahl der ersten Leistungsstufe eine reduzierte Pumpleistung von der Handstückeinheit 1 in der Einweg-Spüldüseneinheit und in der zweiten Leistungsstufe die maximale Pumpleistung von der Handstückeinheit 1 in der Einweg-Spüldüseneinheit hervorgerufen wird, d.h. die entsprechende Frequenz der pulsierenden Schubbewegung entsprechend erhöht oder erniedrigt wird.

Zum Betrieb der Elektromotoreinheit 4 in der ersten Leistungsstufe wird diese beispielsweise mit einer Betriebsspannung von ca. 5 V und in der zweiten Leistungsstufe mit einer Betriebsspannung von ca. 13,5 V versorgt. Die Elektromotoreinheit 4 gibt beispielsweise in der ersten Leistungsstufe eine Leistung von ca. 2,6 Watt und in der zweiten Leistungsstufe eine Leistung von ca. 10,8 Watt ab. Die Drehzahl der Elektromotoreinheit beträgt unter Berücksichtigung dessen in der ersten Leistungsstufe ca. 1350 RPM bzw. in der zweiten Leistungsstufe ca. 4150 RPM. Damit kann unter Verwendung einer eine Pumpmembran aufweisenden Einweg-Spüldüseneinheit in der ersten Leistungsstufe eine Pumpwirkung von ca. 7,5 ml/sec und in der zweiten Leistungsstufe von ca. 15 ml/sec erzielt werden.

In einer weiteren bevorzugten Ausführungsform weist die Handstückeinheit 1 zumindest eine Anzeigeeinheit auf, welche die jeweils ausgewählte Betriebsart und/oder die ausgewählte Leistungsstufe der Elektromotoreinheit anzeigt.

Die Anzeigeeinheit kann entweder in die zweiten und dritten Betätigungsmittel integriert sein oder separat hierzu ausgebildet sein. In einer bevorzugten Ausführungsform ist die Anzeigeeinheit in Form einer Leuchtdiode, einer Lampe oder einer Displayeinheit realisiert.

Die Handstückeinheit 1 ist vorzugsweise wasserdicht und damit re-sterilisierbar ausgebildet.

Die Erfindung wurde voranstehend an einem Ausführungsbeispiel beschrieben. Es versteht sich, dass zahlreiche Modifikationen und Änderungen der Erfindung möglich sind, ohne dass hierdurch der Erfindungsgedanke verlassen wird. So können selbstverständlich externe Stromquellen mit unterschiedlichen Betriebsspannungen Anwendung finden.

### Bezugszeichenliste

- 1: Handstückeinheit
- 2: Gehäuse
- 2.1: erste Gehäusehälfte
- 2.2: zweite Gehäusehälfte
- 3: Antriebsmechanik
- 3.1: stößel- oder pilzartiges Element
- 3.2: Schubstangenelement
- 3.2': Aufnahmeöffnung
- 3.3: Antriebselement
- 3.3': Antriebsstift
- 4: Elektromotoreinheit
- 4.1: Antriebswelle
- 5: Steuereinheit
- 6: erste Anschlusseinheit
- 6.1: kreisförmige Ausnehmung
- 7.1: erste Schaltmittel
- 7.2: zweite Schaltmittel
- 8.1: erste Betätigungsmittel
- 8.2: zweite Betätigungsmittel
- 9: zweite Anschlusseinheit

- M: Mittenachse
- A: Antriebsachse

## Patentansprüche

1. Handstückeinheit für eine Einweg-Spüldüseneinheit zur Reinigung und/oder Spülung von Operationswunden umfassend ein Gehäuse (2) mit einer ersten Anschlusseinheit (6) zur Aufnahme der Einweg-Spüldüseneinheit, einer Antriebsmechanik (3) und einer damit zusammenwirkenden Elektromotoreinheit (4), wobei die Handstückeinheit (1) eine zweite Anschlusseinheit (9) zum Anschluss einer externen elektrischen Energieversorgung aufweist, **dadurch gekennzeichnet, dass** im Gehäuse (2) eine Steuereinheit (5) vorgesehen ist, die zum Betrieb der Elektromotoreinheit (4) in zumindest zwei unterschiedlichen Leistungsstufen und zumindest zwei unterschiedlichen Betriebsarten, und zwar Intervall- oder Dauerbetrieb ausgebildet ist und dass die Handstückeinheit (1) re-sterilisierbar ist.

2. Handstückeinheit nach Anspruch 1, **dadurch gekennzeichnet, dass** die Steuereinheit (5) zur Steuerung der Drehzahl der Elektromotoreinheit (4) vorgesehen ist, und zwar im Bereich zwischen 1200 U/min bis 5000 U/min.

3. Handstückeinheit nach einem der Ansprüche 1 oder 2, **dadurch gekennzeichnet, dass** die Antriebsmechanik (3) zur Umsetzung einer Drehbewegung der Elektromotoreinheit (4) in eine pulsierende Schubbewegung ausgebildet ist.

4. Handstückeinheit nach Anspruch 3, **dadurch gekennzeichnet, dass** die Antriebsmechanik (3) zumindest ein stößel- oder pilzartig ausgebildetes Schubelement (3.1), ein Schubstangenelement (3.2) und ein zylinderförmig ausgebildetes Antriebselement (3.3) umfasst, welches mit der Elektromotoreinheit (4) in Wirkverbindung steht.

5. Handstückeinheit nach Anspruch 3 oder 4, **dadurch gekennzeichnet, dass** Antriebselement (3.3) einen Antriebsstift (3.3') aufweist, der in eine im Schubstangenelementes (3.2) vorgesehene Aufnahmebohrung (3.2') eingreift.

6. Handstückeinheit nach einem der Ansprüche 1 bis 5, **dadurch gekennzeichnet, dass** das Gehäuse (2) aus einem re-sterilisierbar Material hergestellt ist.

7. Handstückeinheit nach einem der Ansprüche 1 bis 6, **gekennzeichnet durch** zumindest ein an die Steuereinheit (5) angeschlossenes Schaltmittel (7.1, 7.2), welches in Wirkverbindung mit einem Betätigungsmittel (8.1, 8,2) steht.

8. Handstückeinheit nach einem der Ansprüche 1 bis 7, **gekennzeichnet durch** zumindest eine Anzeigeneinheit zur Anzeige der Betriebsart und der Leistungsstufe der Elektromotoreinheit (4) vorgesehen ist.

9. Anordnung bestehend aus einer Handstückeinheit (1) nach einem der vorhergehenden Ansprüche, einer Einweg-Spüldüseneinheit sowie einer externen elektrischen Energiequelle, welche vorzugsweise durch ein elektrisches Netzteil mit einem Steckanschluss gebildet ist.

## Claims

1. Handpiece unit for a disposable flushing jet for cleaning and/or flushing operation wounds, comprising a housing (2) with a first connecting unit (6) for receiving the disposable flushing jet unit, a drive mechanism (3) and an electric motor unit (4) interacting therewith, wherein the handpiece unit (1) has a second connecting unit (9) for connecting an external electric energy supply, **characterised in that** in the housing (2) there is a control unit (5) which is designed for operating the electric motor unit (4) in at least two different power stages and at least two different types of operation, namely at intervals or continuous operation, and that the handpiece unit (1) can be re-sterilised.

2. Handpiece unit according to claim 1 **characterised in that** the control unit (5) is provided for controlling the speed of the electric motor unit (4), namely in the range between 1200 rpm and 5000 rpm.

3. Handpiece unit according to one of claims 1 or 2 **characterised in that** the drive mechanism (3) is designed for converting a rotational movement of the electric motor unit (4) into a pulsating translatory movement.

4. Handpiece unit according to claim 3 **characterised in that** the drive mechanism (3) comprises a plunger or mushroom shaped push element (3.1), a push rod element (3.2) and a cylindrical shaped drive element (3.3) which is in active connection with the electric motor unit (4).

5. Handpiece unit according to claim 3 or 4 **characterised in that** the drive element (3.3) has a drive pin (3.3') which engages in a socket bore (3.2') provided in the push rod element (3.2).

6. Handpiece unit according to one of claims 1 to 5 **characterised in that** the housing (2) is made from a re-sterilisable material.

7. Handpiece unit according to one of claims 1 to 6 **characterised by** at least one switching means (7.1, 7.2) connected to the control unit (5) and in active connection with an actuating means (8.1, 8.2).

8. Handpiece unit according to one of claims 1 to 7 **characterised by** at least one indicator unit for indicating the method of operation and the power stage of the electric motor unit (4)

9. Arrangement consisting of a handpiece unit (1) according to one of the preceding claims, a disposable flushing jet unit as well as an external electric energy source which is preferably formed by an electric power supply unit with a plug-type connection.

## Revendications

1. Dispositif formant pièce à main pour un dispositif jetable formant buse de rinçage destiné au nettoyage et/ou au rinçage de plaies opératoires comprenant un boîtier (2) avec un premier dispositif de raccordement (6) destiné à recevoir le dispositif jetable formant buse de rinçage, un mécanisme d'entraînement (3) et un moteur électrique (4) coopérant avec celui-ci, le dispositif formant pièce à main (1) présentant un second dispositif de raccordement (9) destiné au raccordement d'une alimentation externe en énergie électrique, **caractérisé en ce qu'**une unité de commande (5), conçue pour faire fonctionner le moteur électrique (4) selon au moins deux étages de puissance différents et au moins deux types de fonctionnement différents, est prévue dans le boîtier à savoir un fonctionnement intermittent ou un fonctionnement en continu, et le dispositif formant pièce à main (1) peut être restérilisé.

2. Dispositif formant pièce à main selon la revendication 1, **caractérisé en ce que** l'unité de commande (5) est prévue pour commander le régime du moteur électrique (4), dans une plage comprise entre 1 200 tours/minute et 5 000 tours/minute.

3. Dispositif formant pièce à main selon la revendication 1 ou 2, **caractérisé en ce que** le mécanisme d'entraînement (3) est conçu pour convertir un mouvement rotatif du moteur électrique (4) en un mouvement de poussée pulsatoire.

4. Dispositif formant pièce à main selon la revendication 3, **caractérisé en ce que** le mécanisme d'entraînement (3) comprend au moins un élément de poussée (3.1) de type pilon ou champignon, un élément formant bielle (3.2) et un élément d'entraînement (3.3) de forme cylindrique qui est en liaison fonctionnelle avec le moteur électrique (4).

5. Dispositif formant pièce à main selon la revendication 3 ou 4, **caractérisé en ce que** l'élément d'entraînement (3.3) présente une cheville d'entraînement (3.3'), qui s'engage dans un alésage de réception (3.2') prévu dans l'élément formant bielle (3.2).

6. Dispositif formant pièce à main selon l'une quelconque des revendications 1 à 5, **caractérisé en ce que** le boîtier (2) est fabriqué à partir d'un matériau pouvant être restérilisé.

7. Dispositif formant pièce à main selon l'une quelconque des revendications 1 à 6, **caractérisé par** au moins un moyen de commutation (7.1, 7.2) raccordé à l'unité de commande (5), ledit moyen de commutation étant en liaison fonctionnelle avec un moyen d'actionnement (8.1, 8.2).

8. Dispositif formant pièce à main selon l'une quelconque des revendications 1 à 7, **caractérisé en ce qu'**au moins un dispositif d'affichage destiné à l'affichage du type de fonctionnement et de l'étage de puissance du moteur électrique (4) est prévu.

9. Agencement constitué d'un dispositif formant pièce à main (1) selon l'une quelconque des revendications précédentes, d'un dispositif jetable formant buse de rinçage ainsi que d'une source externe d'énergie électrique, qui est formée de manière préférée par un bloc d'alimentation électrique secteur muni d'une prise de raccordement.
